(19) Europäisches Patentamt
European Patent Office
Office européen des brevets

(11) EP 0 543 878 B1

(12) **EUROPÄISCHE PATENTSCHRIFT**

(45) Veröffentlichungstag und Bekanntmachung des Hinweises auf die Patenterteilung:
**08.01.1997 Patentblatt 1997/02**

(21) Anmeldenummer: **91914614.2**

(22) Anmeldetag: **08.08.1991**

(51) Int. Cl.$^6$: **A01N 43/80**, A01N 25/32, C07D 261/04, C07D 275/03

(86) Internationale Anmeldenummer:
**PCT/EP91/01503**

(87) Internationale Veröffentlichungsnummer:
**WO 92/03053 (05.03.1992 Gazette 1992/06)**

(54) **ISOXAZOLINE ODER ISOTHIAZOLINE ENTHALTENDE PFLANZSCHÜTZENDE MITTEL UND NEUE ISOXAZOLINE UND ISOTHIAZOLINE**

PLANT PROTECTING AGENTS CONTAINING ISOXAZOLINE OR ISOTHIAZOLINE AND NEW ISOXAZOLINES AND ISOTHIAZOLINES

AGENTS PHYTOSANITAIRES CONTENANT DE L'ISOXAZOLINE OU DE L'ISOTHIAZOLINE ET NOUVELLES ISOXAZOLINES ET ISOTHIAZOLINES

(84) Benannte Vertragsstaaten:
**DE FR GB**

(30) Priorität: **17.08.1990 DE 4026018**

(43) Veröffentlichungstag der Anmeldung:
**02.06.1993 Patentblatt 1993/22**

(73) Patentinhaber: **Hoechst Schering AgrEvo GmbH
13342 Berlin (DE)**

(72) Erfinder:
- **LÖHER, Heinz-Josef
D-6237 Liederbach (DE)**
- **BAUER, Klaus
D-6450 Hanau (DE)**
- **BIERINGER, Hermann
D-6239 Eppstein (DE)**

(56) Entgegenhaltungen:

| | |
|---|---|
| **EP-A- 0 136 016** | **EP-A- 0 148 795** |
| **EP-A- 0 334 120** | **EP-A- 0 337 263** |
| **WO-A-91/08202** | **JP-A- 621 030** |

- **Chem. Ber., Band. 106, 1973 M. Christl et al.: "Orientierungsphänomene bei Cycloadditionen aliphatischer und aromatischer Nitriloxide an alfa,beta- ungesättigte Carbonester ",**
- **Tetrahedron Letters, Band. 25, Nr. 19, 1984 E. Coutouli-Argyropoulou: "The reaction of triphenylphosphine with arylbromonitromethanes. Formation of arylnitriloxides ",**

EP 0 543 878 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.13.11/3.4

## Beschreibung

Die Erfindung betrifft Safener oder Antidote, die in Kombination mit Herbiziden die Phytotoxizität der Herbizide bei Kulturpflanzen herabsetzen können.

Gegenstand der Erfindung sind kulturpflanzenschützende Mittel, welche Isoxazoline oder Isothiazoline der allgemeinen Formel I oder deren Salze,

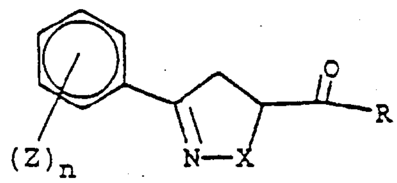

(I)

worin

X ein Sauerstoff- oder Schwefelatom, insbesondere ein Sauerstoffatom,

R OH, SH, Alkoxy, Alkenyloxy, Alkinyloxy, Alkylthio, Alkenylthio, Alkinylthio, Cycloalkyloxy, Cycloalkylthio, wobei die 8 letztgenannten Gruppen unsubstituiert oder ein- oder mehrfach durch Reste aus der Gruppe enthaltend Phenyl, Alkoxy, Alkenyloxy, Alkinyloxy, Benzyloxy, Phenyloxy, Cycloalkyloxy, Alkylthio, Mono- und Dialkylamino, Cyano, Halogen und $NO_2$ substituiert sind,
Benzyloxy, Phenyloxy, Benzylthio, Phenylthio, wobei die 4 letztgenannten Gruppen unsubstituiert oder ein- oder mehrfach durch Rest aus der Gruppe enthaltend Alkyl, Alkenyl, Alkinyl, Halogen, Cyano, $NO_2$, Alkoxy, Alkenyloxy, Alkinyloxy, Alkylthio, Mono- und Dialkylamino, Phenyloxy und Benzyloxy substituiert sind,
Trialkylsilylalkoxy, Aryldialkylsilyloxy, Aralkyldialkylsilyloxy, Diarylalkylsilyloxy, Diaralkylalkylsilyloxy, einen Rest der Formel NR'R', worin die Reste R' für gleiche oder verschiedene Rente aus der Gruppe Alkyl, Alkenyl, Alkinyl und Cycloalkyl stehen, Pyridino, Morpholino, Dialkylmorpholino,
Hydrazino oder
einen Rest der Formel

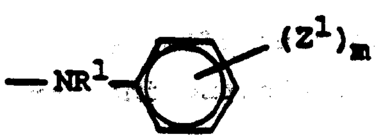

worin $R^1$ ein Wasserstoffatom, Alkyl, Alkenyl oder Alkinyl und die Reste $Z^1$ unabhängig voneinander Halogen, Nitro, Alkyl, Alkenyl, Alkoxy oder Phenoxy und m eine ganze Zahl von 0 bis 5 sind, oder
einen Rest der Formel

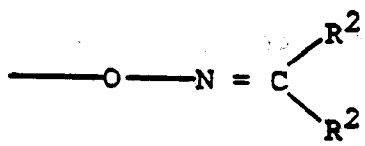

worin die Reste $R^2$ jeweils unabhängig voneinander Alkyl oder gemeinsam mit da sie verknüpfenden C-Atom Cycloalkyl bedeuten, oder

$$- O - CR^3R^3 - CO - R^4$$

worin die $R^3$ unabhängig voneinander H, Alkyl, Alkenyl, Alkinyl, Aryl, Benzyl, Alkoxy, Alkenyloxy, Alkinyloxy oder Phenoxy bedeuten und $R^4$ Wasserstoff, Alkyl Alkenyl, Alkinyl, Aryl oder Benzyl bedeutet, oder
einen Rest der Formel

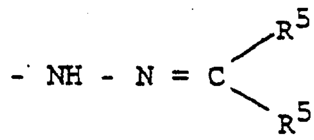

worin die $R^5$ unabhängig voneinander Alkyl, H oder Aryl oder gemeinsam mit dem sie verknüpfenden C-Atom Cycloalkyl bedeuten oder einen Rest der Formel

$$- O - CR^6R^6 - CO - R^7$$

worin die Reste $R^6$ unabhängig voneinander H, Alkyl, Alkenyl, Alkinyl, Aryl, Benzyl, Alkoxy, Alkenyloxy, Alkinyloxy oder Phenoxy bedeuten und $R^7$ die vorstehend für R angegebene Bedeutung hat,

Z Halogen, Nitro, Cyano, $(C_1$-$C_4)$-Alkyl, $(C_1$-$C_4)$-Alkoxy, $(C_1$-$C_4)$-Alkylthio, wobei die Alkyl-, Alkoxy- und Alkylthiogruppen unabhängig voneinander unsubstituiert oder durch ein oder mehrere Halogenatome, insbesondere Fluor oder Chlor, substituiert sind, $(C_3$-$C_6)$-Cycloalkyl, das unsubstituiert oder durch $(C_1$-$C_4)$-Alkyl substituiert ist, Amino, Hydroxymethyl, $(C_1$-$C_4)$-Alkylamino, Di-$(C_1$-$C_4)$-alkylamino, $(C_1$-$C_4)$-Alkoxymethyl, wobei die Alkyl- und Alkoxygruppen in den 3 letztgenannten Resten unabhängig voneinander unsubstituiert oder durch $(C_1$-$C_4)$-Alkyl substituiert sind, Phenyl oder Phenoxy, wobei Phenyl und Phenoxy unabhängig voneinander unsubstituiert oder ein- oder mehrfach durch Reste aus der Gruppe Halogen und Trifluormethyl substituiert sind,

n eine ganze Zahl von 0 bis 5, insbesondere 0 bis 3,

bedeuten, und ein oder mehrere Herbizide sowie gegebenenfalls übliche Formulierungshilfsmittel enthalten.

In der Formel (I) können Alkyl, Alkoxy- und Alkylthioreste sowie die entsprechenden ungesättigten Reste jeweils geradkettig oder verzweigt sein und haben vorzugsweise weniger als 5 C-Atome. Halogen bedeutet Fluor, Chlor, Brom oder Jod, vorzugsweise Fluor, Chlor oder Brom, insbesondere Fluor oder Chlor.

Im Falle Z = OH können die Verbindungen der Formel (I) Salze bilden. Erfindungsgemäß einsetzen lassen sich die in der Landwirtschaft verwendbaren Salze. Als solche kommen beispielsweise Metallsalze wie Alkali- oder Erdalkalisalze, insbesondere Natrium- oder Kaliumsalze, Salze mit Ammonium, Mono-, Di-, Tri- oder Tetra-$(C_1$-$C_4)$alkylammonium oder mit Mono-, Di-, Tri- oder Tetra-$(C_1$-$C_4)$alkanolammonium infrage.

Gegenstand der Erfindung sind insbesondere auch alle Stereoisomeren und deren Gemische, die von der Formel (I) umfaßt, jedoch nicht spezifisch definiert sind. Stereoisomere können vor allem auftreten, wenn ein oder mehrere asymmetrische C-Atome und/oder geeignet substituierte Doppelbindungen in den Verbindungen der Formel (I) vorhanden sind. Die Stereoisomere lassen sich aus racemischen Gemischen nach üblichen Trennmethoden erhalten. Alternativ können Stereoisomere durch Einsatz stereoselektiver Reaktionen unter Verwendung optisch aktiver Ausgangsstoffe selektiv hergestellt werden.

Von besonderem Interesse sind erfindungsgemäße pflanzenschützende oder selektive herbizide Mittel, die eine Verbindung der genannten Formel (I) enthalten, worin

R OH, SH, $(C_1$-$C_4)$-Alkoxy, $(C_2$-$C_4)$-Alkenyloxy, $(C_2$-$C_4)$-Alkinyloxy, $(C_1$-$C_4)$-Alkylthio, $(C_2$-$C_4)$-Alkenylthio, $(C_2$-$C_4)$-Alkinylthio, $(C_3$-$C_8)$-Cycloalkoxy, $(C_3$-$C_8)$-Cycloalkylthio, wobei die 8 letztgenannten Gruppen unsubstituiert oder ein- oder mehrfach durch Reste aus der Gruppe Phenyl, $(C_1$-$C_4)$-Alkoxy, $(C_2$-$C_4)$-Alkenyloxy, $(C_2$-$C_4)$-Alkinyloxy, Benzyloxy, Phenyloxy, $(C_3$-$C_8)$-Cycloalkyloxy, $(C_1$-$C_4)$-Alkylthio, Mono- und Di-$(C_1$-$C_4)$-Alkylamino, Cyano, Halogen und $NO_2$ substituiert sind,
Phenyloxy, Phenylthio, Benzyloxy, Benzylthio, wobei die 4 letztgenannten Gruppen unsubstituiert oder durch 1 bis 3 Reste aus der Gruppe $(C_1$-$C_4)$-Alkyl, $(C_2$-$C_4)$-Alkenyl, $(C_2$-$C_4)$-Alkinyl, Halogen, Cyano, $NO_2$, $(C_1$-$C_4)$-Alkoxy, $(C_2$-$C_4)$-Alkenyloxy, $(C_2$-$C_4)$-Alkinyloxy, $(C_1$-$C_4)$-Alkylthio, Mono- und Di-$(C_1$-$C_4)$-Alkylamino, Phenyloxy und Benzyloxy substituiert sind, oder
einen Rest der Formel -NR'R', worin R' $(C_1$-$C_4)$-Alkyl bedeutet, Pyridino, Morpholino, Dimethylmorpholino, Hydrazino oder einen Rest der Formel

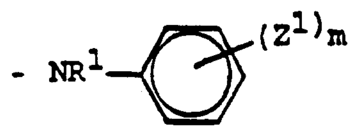

worin $R^1$, H oder ($C_1$-$C_4$)-Alkyl, die Reste $Z^1$ unabhängig voneinander Halogen, Nitro, ($C_1$-$C_4$)-Alkyl, ($C_1$-$C_4$)-Alkoxy oder Phenoxy und m 0 bis 3 bedeuten, oder
einen Rest der Formel -O-N=$CR^2R^2$, worin die $R^2$ ($C_1$-$C_4$)-Alkyl, insbesondere Methyl, oder gemeinsam mit dem sie verknüpfenden C-Atom Cyclohexyl oder Cyclopentyl bedeuten,
oder einen Rest der Formel -O-$CR^3R^3$-CO-$R^4$, worin die $R^3$ unabhängig voneinander für H, ($C_1$-$C_4$)-Alkyl, ($C_2$-$C_4$)-Alkenyl, ($C_2$-$C_4$)-Alkinyl, Phenyl, Benzyl oder
($C_1$-$C_4$)-Alkoxy stehen und $R^4$ für ($C_1$-$C_4$)-Alkyl, ($C_2$-$C_4$)-Alkenyl, ($C_2$-$C_4$)-Alkinyl, Phenyl oder Benzyl steht, oder einen Rest der Formel -NH-N=$CR^5R^5$, worin die $R^5$ unabhängig voneinander H, ($C_1$-$C_4$)-Alkyl oder Phenyl oder gemeinsam mit dem sie verknüpfenden C-Atom Cyclohexyl oder Cyclopentyl bedeuten,
oder einen Rest der Formel -O-$CR^6R^6$-CO-$R^7$, worin die $R^6$ unabhängig voneinander für H, ($C_1$-$C_4$)-Alkyl, ($C_2$-$C_4$)-Alkenyl, ($C_2$-$C_4$)-Alkinyl, Phenyl, Benzyl oder ($C_1$-$C_4$)-Alkoxy stehen und $R^7$ die für R genannte Bedeutung hat, bedeutet.

Aus der Literatur sind die Verbindungen der folgenden Tabelle 1 bekannt, ihre Safenerwirkung wurde jedoch bisher nicht erkannt; vgl. z.B. Chem. Ber., Bd. 106, 3275 (1973), Zh. Obs. Khim., Bd. 52 (8), 1932-3 (1982); Chem. Pharm. Bull., Bd. 28 (11), 3296 (1980); Tetrahedron Lett., Bd. 25 (19), 2029 (1984); Aust. J. Chem., Bd. 37 (6), 1217 (1984); Tetrahedron, Bd. 40 (15), 2985 (1984); J. Chem. Soc. Chem. Commun., Bd. 1984 (22), 1523-14 (1984); J. Org. Chem., Bd. 52, 2137 (1987); J. Org. Chem., Bd. 53, 2238 (1988); Chem. Ber., Bd. 106, 3345 (1973) und JP-A-62-103070 (1987):

Tabelle 1

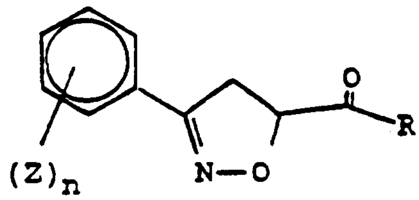

(Ia)

| $(Z)_n$ | R |
|---|---|
| H | $-OCH_3$ |
| 3-$CH_3$ | $-OCH_3$ |
| 4-$CH_3$ | $-OCH_3$ |
| 2,3-$Cl_2$ | $-OCH_3$ |
| 2,4-$Cl_2$ | $-OCH_3$ |

Fortsetzung Tabelle 1

| $(Z)_n$ | R |
|---|---|
| $4,5-Cl_2$ | $-OCH_3$ |
| $3-OC_6H_5$ | $-OCH_3$ |
| $4-OCF_2Br$ | $-OCH_3$ |
| $4-OCF_3$ | $-OCH_3$ |
| $2-Cl, 5-NO_2$ | $-OCH_3$ |
| 4-Phenoxyphenyl | $-OCH_3$ |
| $3-CF_3$ | $-OCH_3$ |
| 3-Cl, 4-F | $-OCH_3$ |
| $2,6-Cl_2$ | $-OCH_3$ |
| $3-OCF_2H$ | $-OCH_3$ |
| 3-F | $-OCH_3$ |
| $4-CF_3$ | $-OCH_3$ |
| $5,6-Cl_2$ | $-OCH_3$ |
| $4,6-Cl_2$ | $-OCH_3$ |
| 3-Br | $-OCH_3$ |
| 4-CN | $-OCH_3$ |
| 4-F | $-OCH_3$ |
| 4-Br | $-OCH_3$ |
| $3-NO_2$ | $-OCH_3$ |
| $3,4-Cl_2$ | $-OCH_3$ |
| $4-OCHF_2$ | $-OCH_3$ |
| 2-Cl | $-OCH_3$ |
| $4-NO_2$ | $-OCH_3$ |
| $4-OCH_3$ | $-OCH_3$ |
| $3,4-Cl_2$ | OH |
| $4-CF_3$ | OH |
| 4-Cl | OH |
| H | OH |
| H | $-OC_2H_5$ |
| 4-Cl | $-OC_2H_5$ |
| 4-Cl | $-O-isoC_3H_7$ |

Außerdem beschreiben US-A-4889551, Chem. Ber. 106 (1973) 3348 und Tetrahedron Lett. 1984, 2029-2030 einzelne Verbindungen der Formel (I), ohne deren Safenereigenschaften zu erwähnen. Gegenstand der Erfindung sind daher auch die bisher nicht bekannten Verbindungen der oben definierten Formel (I) oder deren Salze. Diese sind Verbindungen der Formel (I) oder deren Salze, ausgenommen Verbindungen der Formel (I), in denen X = Sauerstoffatom und

a) R = OH oder ($C_1$-$C_5$)-Alkoxy und $(Z)_n$ = einen einzelnen Rest in 2-, 3- oder 4-Position am Phenylring aus der Gruppe F, Cl, Br, CN, $OCHF_2$, $CF_3$ und $NO_2$ oder $(Z)_n$ = 3-Phenoxy, 2,3-, 2,4-, 2,5- oder 3,4-$Cl_2$ oder 2,4-, 3,4- oder 3,5-$Br_2$ oder 3-Cl-4-F, 5-Cl-2-$NO_2$ oder 2-Cl-5-$NO_2$ oder
b) R = $OCH_3$ und $(Z)_n$ = H, 3-$CH_3$, 4-$CH_3$, 2,6-, 4,6- oder 5,6-$Cl_2$, 4-$OCF_2Br$, 4-$OCF_3$, 4-Phenoxy, 4-Phenoxyphenyl, 2,4,6-$(OCH_3)_3$ oder 2,4,6-$(CH_3)_3$ oder
c) R = OH oder $OC_2H_5$ und $(Z)_n$ = H bedeuten.

Die bekannten und die bisher noch nicht bekannten Verbindungen der Formel (I) können nach oder analog den in der genannten Literatur beschriebenen Verfahren hergestellt werden. Beispielsweise erhält man Verbindungen der Formel (I), indem man ein Acrylsäurederivat der Formel (II)

$$H_2C = CH - CO - R \qquad (II)$$

mit einer Verbindung der Formel (III)

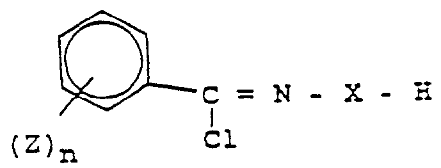

(III)

umsetzt, wobei in den Formeln (II) und (III) R, Z, n und X die genannten Bedeutungen haben. Die Umsetzung wird vorzugsweise in einem aprotischen, dipolaren organischen Lösungsmittel wie Ether bei -10°C bis zum Siedepunkt des Lösungsmittels und in Gegenwart einer organischen Base wie Triethylamin und Pyridin oder einer anorganischen Base wie Kalium- und Natriumcarbonat oder Natriumbicarbonat durchgeführt.

Die Verbindungen der Formel (II) und (III) sind bekannt oder lassen sich nach allgemein bekannten Verfahren herstellen (siehe beispielsweise J. Am. Chem. Soc., Bd. 46, 731 (1924); Angew. Chem., Bd. 75, 604 (1963)).

Die Verbindungen der Formel (I) reduzieren oder unterbinden phytotoxische Nebenwirkungen von Pflanzenschutzmitteln, insbesondere von Herbiziden, die bei deren Einsatz in Nutzpflanzenkulturen auftreten können.

Die Verbindungen der Formel (I) können nacheinander oder zusammen mit den herbiziden Wirkstoffen ausgebracht werden. Sie sind dann in der Lage, schädliche Nebenwirkungen der Herbizide bei Kulturpflanzen zu vermindern oder völlig aufzuheben, ohne die Wirksamkeit dieser Herbizide gegen Schadpflanzen zu beeinträchtigen.

Hierdurch kann das Einsatzgebiet herkömmlicher Pflanzenschutzmittel ganz erheblich erweitert werden. Solche Verbindungen, die die Eigenschaft besitzen, Kulturpflanzen gegen phytotoxische Schäden durch Herbizide zu schützen, werden "Antidote" oder "Safener" genannt.

Herbizide, deren phytotoxische Nebenwirkungen mittels der Verbindungen der Formel (I) herabgesetzt werden können, sind z.B. Carbamate, Thiocarbamate, Halogenacetanilide, substituierte Phenoxy-, Naphthoxy- und Phenoxyphenoxycarbonsäurederivate sowie Heteroaryloxyphenoxycarbonsäurederivate wie Chinolyloxy-, Chinoxalyloxy-, Pyridyloxy-, Benzoxazolyloxy-, Benzthiazolyloxy-phenoxy-carbonsäureester und ferner Dimedonoximabkömmlinge. Bevorzugt hiervon sind Phenoxyphenoxy- und Heteroaryloxyphenoxyalkancarbonsäureester. Als Ester kommen hierbei insbesondere niedere Alkyl-, Alkenyl und Alkinylester in Frage.

Beispielsweise seien, ohne daß dadurch eine Beschränkung erfolgen soll, folgende Herbizide genannt:

A) Herbizide vom Typ der Phenoxyphenoxy- und Heteroaryloxyphenoxycarbonsäure-($C_1$-$C_4$)alkyl-, -($C_2$-$C_4$)alkenyl- und -($C_3$-$C_4$)alkinylester wie 2-(4-(2,4-Dichlorphenoxy)-phenoxy)-propionsäuremethylester, 2-(4-(4-Brom-2-chlorphenoxy)-phenoxy)-propionsäuremethylester, 2-(4-(4-Trifluormethylphenoxy)-phenoxy)-propionsäuremethylester, 2-(4-(2-Chlor-4-trifluormethylphenoxy)-phenoxy)-propionsäuremethylester, 2-(4-(2,4-Dichlorbenzyl)-phenoxy)-propionsäuremethylester, 4-(4-(4-Trifluormethylphenoxy)-phenoxy)-pent-2-en-säureethylester, 2-(4-(3,5-Dichlorpyridyl-2-oxy)-phenoxy)-propionsäureethylester, 2-(4-(3,5-Dichlorpyridyl-2-oxy)-phenoxy)-propionsäurepropargylester, 2-(4-(6-Chlorbenzoxazol-2-yl-oxy)-phenoxy)-propionsäureethylester, 2-(4-(3-Chlor-5-trifluormethyl-2-pyridyloxy)-phenoxy)-propionsäuremethylester, 2-(4-(5-Trifluormethyl-2-pyridyloxy)-phenoxy)-propionsäurebutylester, 2-(4-(3-Fluor-5-chlorpyridyl-2-oxy)-phenoxy)-propionsäuremethylester, 2-(4-(3-Fluor-5-chloropyridyl-2-oxy)-phenoxy)-propionsäurepropargylester, 2-(4-(6-Chlor-2-chinoxalyloxy)phenoxy)-propionsäuremethylester, 2-(4-(6-Fluor-2-chinoxalyloxy)phenoxy)-propionsäuremethylester, 2-(4-(6-Chlor-2-chinolyloxy)-phenoxy)-propionsäuremethylester, 2-(4-(5-Chlor-3-fluorpyridin-2-yloxy)-phenoxy)-thiopropionsäure-8-methoxycarbonylmethylester

B) Chloracetanilid-Herbizide wie N-(3'-Methoxyprop-2'-yl)-methyl-6-ethyl-chloracetanilid, N-(3-Methyl-1,2,4-oxdiazol-5-yl-methyl)-chloressigsäure-2,6-dimethylanilid,

C) Thiocarbamate wie S-Ethyl-N,N-dipropylthiocarbamat oder S-Ethyl-N,N-diisobutylthiocarbamat

D) Dimedon-Derivate wie
Methyl-3-(1-allyloxyimino)butyl-4-hydroxy-6,6-dimethyl-2-oxocyclohex-3-encarboxylat
2-(N-Ethoxybutyrimidoyl)-5-(2-ethylthiopropyl)-3-hydroxy-2-cyclohexen-1-on,
2-(N-Ethoxybutyrimidoyl)-5-(2-phenylthiopropyl)-3-hydroxy-2-cyclohexen-1-on,
2-(1-Allyloxyiminobutyl)-4-methoxycarbonyl-5,5-dimethyl-3-oxocyclohexenol,
2-(1-(3-Chlorallyloxy)-iminobutyl)-5-(2-ethylthio)propyl)-3-hydroxy-cyclohex-2-enon
2-(1-(Ethoxyimino)-butyl)-3-hydroxy-5-(thian-3-yl)-cyclohex-2-enon oder
2-(1-Ethoxyiminopropyl)-5-(2,4,6-trimethylphenyl)-3-hydroxy-2-cyclohexen-1-on.

Das Mengenverhältnis Safener:Herbizid kann innerhalb weiter Grenzen, im Bereich zwischen 1:10 und 10:1, insbesondere zwischen 2:1 und 1:10, schwanken. Die jeweils optimalen Mengen an Herbizid und Safener sind abhängig vom Typ des verwendeten Herbizids oder vom verwendeten Safener sowie von der Art des zu behandelnden Pflanzenbestandes und lassen sich von Fall zu Fall durch entsprechende Versuche ermitteln.

Haupteinsatzgebiete für die Anwendung der Safener sind vor allem Getreidekulturen (Weizen, Roggen, Gerste, Hafer) Reis, Mais, Sorghum, aber auch Baumwolle, Zuckerrübe, Zuckerrohr und Sojabohne.

Die Safener der Formel (I) können je nach ihren Eigenschaften zur Vorbehandlung des Saatgutes der Kulturpflanze (Beizung der Samen) verwendet werden oder vor der Saat in die Saatfurchen eingebracht werden oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Die benötigten Aufwandmengen der Verbindungen der Formel (I) können je nach Indikation und verwendetem Herbizid innerhalb weiter Grenzen schwanken und variieren im allgemeinen zwischen 0,01 und 10 kg Wirkstoff je Hektar.

Gegenstand der vorliegenden Erfindung ist deshalb auch ein Verfahren zum Schutz von Kulturpflanzen vor phytotoxischen Nebenwirkungen von Herbiziden, das dadurch gekennzeichnet ist, daß eine wirksame Menge einer Verbindung der Formel (I) vor, nach oder gleichzeitig mit dem Herbizid appliziert wird.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die Verbindungen der Formel (I) oder deren Kombination mit einem oder mehreren der genannten Herbizide bzw. Herbizidgruppen können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), emulgierbare Konzentrate (EC), wäßrige Lösungen (SL), konzentrierte Emulsionen wie Öl-in-Wasser-und Wasser-in-Öl-Emulsionen (EW), versprühbare Lösungen oder Emulsionen, Dispersionen auf Öl- oder Wasserbasis (SC), Stäubemittel (DP), Beizmittel, Boden- bzw. Streugranulate (FG), wasserdispergierbare Granulate (WG), ULV-Formulierungen, Mikrokapseln oder Wachse.

Diese einzelnen Formulierungstypen sind im Prinzip bekannt und werden beispielsweise beschrieben in: Winnakker-Küchler, "Chemische Technologie", Band 7, C. Hauser Verlag München, 4. Aufl. 1986; van Valkenburg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Intruduction to Clay Colloid Chemistry"; 2nd Ed., J. Wiley & Sons, N.Y., Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technolgie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole oder Fettamine,

Alkansulfonate oder Alkylarylsulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymerisate) Alkylpolypolyglykolether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde.

Boden- bzw. Streu-Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0,1 bis 99 Gewichtsprozent, insbesondere 0,1 bis 95 Gew.-%, Wirkstoff der Formel (I), oder Wirkstoffgemisch Antidot/Herbizid, 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 80 Gew.-%, vorzugsweise 5 bis 80 Gew.-% betragen. Staubförmige Formulierungen enthalten meistens 1 bis 25 Gew.-%, vorzugsweise 5 bis 20 Gew.-% an Wirkstoff, versprühbare Lösungen etwa 0,2 bis 25 Gew.-%, vorzugsweise 2 bis 20 Gew.-% Wirkstoff. Bei wasserdispergierbaren Granulaten hängt der Wirkstoffgehalt zum Teil davon ab, ob die wirksame Verbindung flüssig oder fest vorliegt. Im allgemeinen liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate, sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids u.a. variiert die erforderliche Aufwandmenge der Verbindungen der Formel (I).

Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Folgende Beispiele dienen zur Erläuterung der Erfindung:

A) Formulierungsbeispiele

a) Ein Stäubmittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühie auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexanon als Lösemittel und 10 Gew.-Teilen oxethyliertes Nonylphenol als Emulgator.

e) Ein in Wasser dispergierbares Granulat wird erhalten indem man

75 Gewichtsteile einer Verbindung der Formel I,
10 Gewichtsteile ligninsulfonsaures Calcium,
5 Gewichtsteile Natriumlaurylsulfat,
3 Gewichtsteile Polyvinylalkohol und
7 Gewichtsteile Kaolin

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten indem man

25 Gewichtsteile einer Verbindung der Formel (I),
5 Gewichtsteile 2,2'-Dinaphthylmethan-6,6'-disulfonsaures Natrium,
2 Gewichtsteile oleoylmethyltaurinsaures Natrium,
1 Gewichtsteil Polyvinylalkohol,
17 Gewichtsteile Calciumcarbonat und
50 Gewichtsteile Wasser

auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet.

g) Analog den vorstehenden Formulierungen erhält man Formulierungen selektiver herbizider Mittel, wenn man anstelle des Wirkstoffes der Formel (I) ein Gemisch aus
Wirkstoff der Formel (I) mit einem Herbizid wie z.B. Fenoxaprop-ethyl einsetzt.

B) Chemische Beispiele:

3-(2-Methoxyphenyl)-2-isoxazolin-5-carbonsäureethylester (Beispiel 78, s. Tabelle 2)

50 ml Acrylsäureethylester und 4,04 g Triethylamin werden vorgelegt. Dazu tropft man 7,4 g 2-Methoxy-phenylhydroxamsäurechlorid in 200 ml Ether bei 0°C. Nach dreistündigem Rühren bei Raumtemperatur werden 50 ml Wasser zugegeben und das Gemisch mit Ether extrahiert. Nach Trocknen über $MgSO_4$ wird der Ether abdestilliert und das Rohprodukt über eine Kieselgel-Säule (Laufmittel: n-Heptan:Essigester = 8:2) gereinigt. So werden 8,4 g (84 % d.Th.) des gemischten Produkts mit einem Brechungsindex von $n_D^{20}$ = 1.544 erhalten.

Die Derivate der folgenden Tabelle 2 werden auf analogem Weg erhalten.

Tabelle 2:

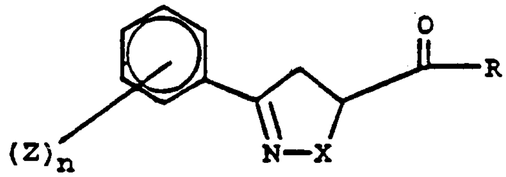

| Beisp. Nr. | $(Z)_n$ | R | X | mp./$n_D^{20}$ |
|---|---|---|---|---|
| 1 | H | OH | O | 197°C |
| 2 | " | $OCH_3$ | " | 73°C |
| 3 | " | $OC_2H_5$ | " | 1.5457 |
| 4 | " | n-$OC_3H_2$ | " | |
| 5 | " | i-$OC_3H_7$ | " | 1.5335 |
| 6 | " | n-$OC_4H_9$ | " | |
| 7 | " | O-$CH_2CO_2C_2H_5$ | " | |
| 8 | " | O-$C_6H_5$ | " | |
| 9 | " | O-$CH_2C_6H_5$ | " | |
| 10 | " | $OCH_2CH=CH_2$ | " | |
| 11 | " | $OCH_2C≡CH$ | " | |
| 12 | " | O-$CH_2Si(CH_3)_3$ | " | |
| 13 | " | $O^-K^+$ | " | |
| 14 | 2-Cl | OH | " | |
| 15 | " | $OCH_3$ | " | 1.5609 |
| 16 | " | $OC_2H_5$ | " | 1.5519 |
| 17 | " | n-$OC_3H_7$ | " | |
| 18 | " | i-$OC_3H_7$ | " | |
| 19 | " | n-$OC_4H_9$ | " | |
| 20 | " | O-$CH_2CO_2C_2H_5$ | " | |
| 21 | " | $OC_6H_5$ | " | |
| 22 | " | $OCH_2C_6H_5$ | " | |
| 23 | " | O-$CH_2CH=CH_2$ | " | |
| 24 | " | O-$CH_2C≡CH$ | " | |
| 25 | " | O-$CH_2Si(CH_3)_3$ | " | |
| 26 | " | $O^-$ $K^+$ | " | |

Fortsetzung von Tabelle 2

| Beisp. Nr. | $(Z)_n$ | R | X | mp./$n_D^{20}$ |
|---|---|---|---|---|
| 27 | 4-Cl | OH | O | |
| 28 | " | $OCH_3$ | " | 75°C |
| 29 | " | $OC_2H_5$ | " | 68°C |
| 30 | " | n-$OC_3H_7$ | " | |
| 31 | " | i-$OC_3H_7$ | " | |
| 32 | " | n-$OC_4H_9$ | " | |
| 33 | " | O-$CH_2CO_2C_2H_5$ | " | |
| 34 | " | O-$C_6H_5$ | " | |
| 35 | " | O-$CH_2C_6H_5$ | " | |
| 36 | " | O-$CH_2CH{=}CH_2$ | " | |
| 37 | " | O-$CH_2C{\equiv}CH$ | " | |
| 38 | " | O-$CH_2Si(CH_3)_3$ | " | |
| 39 | 2,4-$Cl_2$ | OH | " | |
| 40 | " | $OCH_3$ | " | 58°C |
| 41 | " | $OC_2H_5$ | " | 64°C |
| 42 | " | $OC_3H_7$ (n) | " | |
| 43 | " | $OC_3H_7$ (iso) | " | |
| 44 | " | $OC_4H_9$ (n) | " | |
| 45 | " | O-$CH_2CO_2C_2H_5$ | " | 38°C |
| 46 | " | $OC_6H_5$ | " | |
| 47 | " | $OCH_2C_6H_5$ | " | |
| 48 | " | O-$CH_2CH{=}CH_2$ | " | |
| 49 | " | O-$CH_2C{\equiv}CH$ | " | |
| 50 | " | O-$CH_2Si(CH_3)_3$ | " | |
| 51 | " | $OCH_2CO_2CH_3$ | " | Oel |
| 52 | 2,6-$Cl_2$ | OH | " | 140°C |
| 53 | " | $OCH_3$ | " | 1.5541 |
| 54 | " | $OC_2H_5$ | " | 1.5372 |
| 55 | " | $OC_3H_7$ (n) | " | |
| 56 | " | $OC_3H_7$ (iso) | " | |
| 57 | " | $OC_4H_9$ (n) | " | 1.5302 |

Fortsetzung von Tabelle 2

| Beisp. Nr. | $(Z)_n$ | R | X | mp./$n_D^{20}$ |
|---|---|---|---|---|
| 58 | 2,6-$Cl_2$ | O-$CH_2CO_2C_2H_5$ | O | |
| 59 | " | O-$C_6H_5$ | " | |
| 60 | " | O-$CH_2C_6H_5$ | " | |
| 61 | " | O-$CH_2CH$=$CH_2$ | " | 1.5472 |
| 62 | " | O-$CH_2C{\equiv}CH$ | " | 67°C |
| 63 | " | O-$CH_2Si(CH_3)_3$ | " | |
| 64 | 4-$OCH_3$ | OH | " | |
| 65 | " | $OCH_3$ | " | 75°C |
| 66 | " | $OC_2H_5$ | " | 1.5535 |
| 67 | " | $OC_3H_7$ (n) | " | |
| 68 | " | $OC_3H_7$ (iso) | " | |
| 69 | " | $OC_4H_9$ (n) | " | |
| 70 | " | O-$CH_2CO_2C_2H_5$ | " | |
| 71 | " | $OC_6H_5$ | " | |
| 72 | " | $OCH_2C_6H_5$ | " | |
| 73 | " | O-$CH_2CH$=$CH_2$ | " | |
| 74 | " | O-$CH_2C{\equiv}CH$ | " | |
| 75 | " | O-$CH_2Si(CH_3)_3$ | " | |
| 76 | 2-$OCH_3$ | OH | " | |
| 77 | " | $OCH_3$ | " | Oel |
| 78 | " | $OC_2H_5$ | " | 1.544 |
| 79 | " | $OC_3H_7$ (n) | " | |
| 80 | " | $OC_3H_7$ (iso) | " | |
| 81 | " | $OC_4H_9$ (n) | " | |
| 82 | " | O-$CH_2CO_2C_2H_5$ | " | |
| 83 | " | O-$C_6H_5$ | " | |
| 84 | " | O-$CH_2C_6H_5$ | " | |
| 85 | " | O-$CH_2CH$=$CH_2$ | " | |
| 86 | " | O-$CH_2C{\equiv}CH$ | " | |
| 87 | " | O-$CH_2Si(CH_3)_3$ | " | |

Fortsetzung von Tabelle 2

| Beisp. Nr. | $(Z)_n$ | R | X | mp./$n_D^{20}$ |
|---|---|---|---|---|
| 88 | 2-$CH_3$ | OH | " | |
| 89 | " | $OCH_3$ | " | |
| 90 | " | $OC_2H_5$ | " | |
| 91 | " | $OC_3H_7$ (n) | " | |
| 92 | " | $OC_3H_7$ (iso) | " | |
| 93 | " | $OC_4H_9$ (n) | " | |
| 94 | " | O-$CH_2CO_2C_2H_5$ | " | |
| 95 | " | $OC_6H_5$ | " | |
| 96 | " | $OCH_2C_6H_5$ | " | |
| 97 | " | O-$CH_2CH{=}CH$ | " | |
| 98 | " | O-$CH_2C{\equiv}CH$ | " | |
| 99 | " | O-$CH_2Si(CH_3)_3$ | " | |
| 100 | H | $OCH_3$ | S | |
| 101 | " | $OC_2H_5$ | " | |
| 102 | 4-Cl | $OCH_3$ | " | |
| 103 | " | $OC_2H_5$ | " | |
| 104 | 2,4-$Cl_2$ | $OCH_3$ | " | |
| 105 | " | $OC_2H_5$ | " | |
| 106 | 2,6-$Cl_2$ | $OCH_3$ | " | |
| 107 | " | $OC_2H_5$ | " | |
| 108 | 4-$OCH_3$ | $OCH_3$ | " | |
| 109 | " | $OC_2H_5$ | " | |
| 110 | 2-$OCH_3$ | $OCH_3$ | " | |
| 111 | " | $OC_2H_5$ | " | |
| 112 | 2-$CH_3$ | $OCH_3$ | " | |
| 113 | " | $OC_2H_5$ | " | |
| 114 | 2-Cl | $N(CH_3)_2$ | O | |
| 115 | " | $NHNH_2$ | " | |
| 116 | " | $NH_2$ | " | |

Fortsetzung von Tabelle 2

| Beisp. Nr. | $(Z)_n$ | R | X | mp./$n_D^{20}$ |
|---|---|---|---|---|
| 117 | 2,4-$Cl_2$ | $N(CH_3)_2$ | O | |
| 118 | " | $NHNH_2$ | " | |
| 119 | " | $NH_2$ | " | |
| 120 | 4-Cl | $N(CH_3)_2$ | " | |
| 121 | " | $NHNH_2$ | " | |
| 122 | " | $NH_2$ | " | |

C) Biologische Beispiele

Beispiel 1

Weizen und Gerste wurden im Gewächshaus in Plastiktöpfen bis zum 3-4 Blattstadium herangezogen und dann nacheinander mit erfindungsgemäßen Verbindungen und Herbiziden im Nachauflaufverfahren behandelt. Die Herbizide und die Verbindungen der Formel (I) wurden dabei in Form wäßriger Suspensionen bzw. Emulsionen mit einer Wasseraufwandmenge von umgerechnet 600 bis 800 l/ha ausgebracht. 3-4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Die Bewertung erfolgte in Prozentwerten im Vergleich zu unbehandelten Kontrollen.

Die Ergebnisse aus Tabelle 3 veranschaulichen, daß die erfindungsgemäßen Verbindungen starke Herbizidschäden an Kulturpflanzen effektiv reduzieren können.

Selbst bei starken Überdosierungen des Herbizids H werden bei den Kulturpflanzen auftretende schwere Schädigungen deutlich reduziert und geringere Schäden völlig aufgehoben. Mischungen aus Herbiziden und erfindungsgemäßen Verbindungen eignen sich deshalb in ausgezeichneter Weise zur selektiven Unkrautbekämpfung in Getreidekulturen.

Tabelle 3

| Safenerwirkung der erfindungsgemäßen Verbindungen | | | |
|---|---|---|---|
| Bsp. Nr. | kg a.i./ha | TRAE | HOVU |
| H | 2,0 | 80 | - |
| | 0,2 | - | 85 |
| H+40 | 2,0 + 1,25 | 20 | - |
| H+40 | 0,2 + 1,25 | - | 30 |
| H+41 | 2,0 + 1,25 | 25 | - |
| H+41 | 0,2 + 1,25 | - | 35 |

Abkürzungen:
TRAE = Triticum aestivum
HOVU = Hordeum vulgare
a.i. = Aktivsubstanz
H = 2-(4-(6-Chlorbenzoxazol-2-yloxy)phenoxypropionsäureethylester (Fenoxaprop-ethyl)

## Patentansprüche

1. Kulturpflanzenschützende Mittel, dadurch gekennzeichnet, daß sie eine Verbindung der Formel (I) oder deren Salze

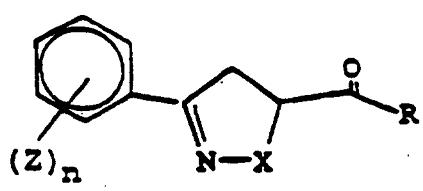

(I)

worin

X ein Sauerstoff- oder Schwefelatom,

R OH, SH, Alkoxy, Alkenyloxy, Alkinyloxy, Alkylthio, Alkenylthio, Alkinylthio, Cycloalkyloxy, Cycloalkylthio, wobei die 8 letztgenannten Gruppen unsubstituiert oder ein- oder mehrfach durch Reste aus der Gruppe enthaltend Phenyl, Alkoxy, Alkenyloxy, Alkinyloxy, Benzyloxy, Phenyloxy, Cycloalkyloxy, Alkylthio, Mono- und Dialkylamino, Cyano, Halogen und $NO_2$ substituiert sind,
Benzyloxy, Phenyloxy, Benzylthio, Phenylthio, wobei die 4 letztgenannten Gruppen unsubstituiert oder ein- oder mehrfach durch Rest aus der Gruppe enthaltend Alkyl, Alkenyl, Alkinyl, Halogen, Cyano, $NO_2$, Alkoxy, Alkenyloxy, Alkinyloxy, Alkylthio, Mono- und Dialkylamino, Phenyloxy und Benzyloxy substituiert sind, Trialkylsilylalkoxy, Aryldialkylsilyloxy, Aralkyldialkylsilyloxy, Diarylalkylsilyloxy, Diaralkylalkylsilyloxy, einen Rest der Formel NR'R', worin die Reste R' für gleiche oder verschiedene Reste aus der Gruppe Alkyl, Alkenyl, Alkinyl und Cycloalkyl stehen, Pyridino, Morpholino, Dialkylmorpholino, Hydrazino, oder
einen Rest der Formel

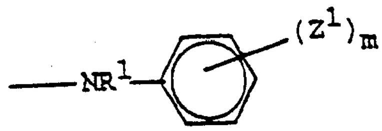

worin $R^1$ ein Wasserstoffatom, Alkyl, Alkenyl oder Alkinyl und die Reste $Z^1$ unabhängig voneinander Halogen, Nitro, Alkyl, Alkenyl, Alkoxy oder Phenoxy und m eine ganze Zahl von 0 bis 5 sind, oder einen Rest der Formel

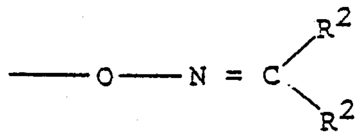 ,

worin die beste $R^2$ jeweils unabhängig voneinander Alkyl oder gemeinsam mit dem sie verknübfenden C-Atom Cycloalkyl bedeuten, oder

$$- O - CR^3R^3 - CO - R^4$$

worin die $R^3$ unabhängig voneinander H, Alkyl, Alkenyl, Alkinyl, Aryl,Benzyl, Alkoxy, Alkenyloxy, Alkinyloxy oder Phenoxy bedeuten und $R^4$ Wasserstoff, Alkyl, Alkenyl, Alkinyl, Aryl oder Benzyl bedeutet, oder einen Rest der Formel

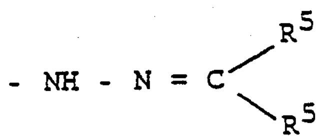

worin die $R^5$ unabhängig voneinander Alkyl, H oder Aryl oder gemeinsam mit dem sie verknüpfenden C-Atom Cycloalkyl bedeuten oder einen Rest der Formel

$$- O - CR^6R^6 - CO - R^7$$

worin die Rente $R^6$ unabhängig voneinander H, Alkyl, Alkenyl, Alkinyl, Aryl, Benzyl, Alkoxy, Alkenyloxy, Alkinyloxy oder Phenol bedeuten umd $R^7$ die vorstehend für R angegebene Bedeutung hat,

Z Halogen, Nitro, Cyano, $(C_1-C_4)$-Alkyl, $(C_1-C_4)$-Alkoxy, $(C_1-C_4)$-Alkylthio, wobei die Alkyl-, Alkoxy- und Alkylthiogruppen unabhängig voneinander unsubstituiert oder durch ein oder mehrere Halogenatome substituiert sind,
$(C_3-C_6)$-Cycloalkyl, das unsubstituiert oder durch $(C_1-C_4)$-Alkyl substituiert ist, Amino, Hydroxymethyl, $(C_1-C_4)$-Alkylamino, Di-$(C_1-C_4)$-alkylamino, $(C_1-C_4)$-Alkoxymethyl, wobei die Alkyl- und Alkoxygruppen in den 3 letztgenannten Resten unabhängig voneinander unsubstituiert oder durch $(C_1-C_4)$-Alkyl substituiert sind, Phenyl oder Phenoxy, wobei Phenyl und Phenoxy unabhängig voneinander unsubstituiert oder ein- oder mehrfach durch Reste aus der Gruppe Halogen und Trifluormethyl substituiert sind,

n eine ganze Zahl von 0 bis 5,

bedeuten, und ein oder mehrere Herbizide sowie gegebenenfalls übliche Formulierungshilfsmittel enthalten.

**2.** Mittel nach Anspruch 1, dadurch gekennzeichnet, daß

R OH, SH, $(C_1-C_4)$-Alkoxy, $(C_2-C_4)$-Alkenyloxy, $(C_2-C_4)$-Alkinyloxy, $(C_1-C_4)$-Alkylthio, $(C_2-C_4)$-Alkenylthio, $(C_2-C_4)$-Alkinylthio, $(C_3-C_8)$-Cycloalkoxy, $(C_3-C_8)$-Cycloalkylthio, wobei die 8 letztgenannten Gruppen unsubstituiert oder ein- oder mehrfach durch Reste aus der Gruppe Phenyl, $(C_1-C_4)$-Alkoxy, $(C_2-C_4)$-Alkenyloxy, $(C_2-C_4)$-Alkinyloxy, Benzyloxy, Phenyloxy, $(C_3-C_8)$-Cycloalkyloxy, $(C_1-C_4)$-Alkylthio, Mono- und Di-$(C_1-C_4)$-Alkylamino, Cyano, Halogen und $NO_2$ substituiert sind,
Phenyloxy, Phenylthio, Benzyloxy, Benzylthio wobei die 4 letztgenannten Gruppen unsubstituiert oder durch 1 bis 3 Reste aus der Gruppe $(C_1-C_4)$-Alkyl, $(C_2-C_4)$-Alkenyl, $(C_2-C_4)$-Alkinyl, Halogen, Cyano, $NO_2$, $(C_1-C_4)$-Alkoxy, $(C_2-C_4)$-Alkenyloxy, $(C_2-C_4)$-Alkinyloxy, $(C_1-C_4)$-Alkylthio, Mono- und Di-$(C_1-C_4)$-Alkylamino, Phenyloxy und Benzyloxy substituiert sind, oder

einen Rest der Formel -NR'R', worin R' ($C_1$-$C_4$)-Alkyl bedeutet, Pyridino, Morpholino, Dimethylmorpholino, Hydrazino, oder einen Rest der Formel

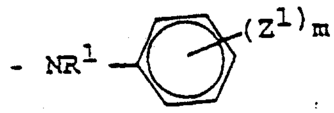

worin $R^1$, H oder ($C_1$-$C_4$)-Alkyl, die Reste $Z^1$ unabhängig voneinander Halogen, Nitro, ($C_1$-$C_4$)-Alkyl, ($C_1$-$C_4$)-Alkoxy oder Phenoxy und m 0 bis 3 bedeuten, oder
einen Rest der Formel -O-N=$CR^2R^2$, worin die $R^2$ ($C_1$-$C_4$)-Alkyl, insbesondere Methyl, oder gemeinsam mit dem sie verknüpfenden C-Atom Cyclohexyl oder Cyclopentyl bedeuten,
oder einen Rest der Formel -O-$CR^3R^3$-CO-$R^4$-, worin die $R^3$ unabhängig voneinander für H, ($C_1$-$C_4$)-Alkyl, ($C_2$-$C_4$)-Alkenyl, ($C_2$-$C_4$)-Alkinyl, Phenyl, Benzyl oder ($C_1$-$C_4$)-Alkoxy stehen und $R^4$ für ($C_1$-$C_4$)-Alkyl, ($C_2$-$C_4$)-Alkenyl, ($C_2$-$C_4$)-Alkinyl, Phenyl oder Benzyl steht, oder
einen Rest der Formel -NH-N=$CR^5R^5$, worin die $R^5$ unabhängig voneinander H, ($C_1$-$C_4$)-Alkyl oder Phenyl oder gemeinsam mit dem sie verknüpfenden C-Atom Cyclohexyl oder Cyclopentyl bedeuten,
oder einen Rest der Formel -O-$CR^6R^6$-CO-$R^7$, worin die $R^6$ unabhängig voneinander für H, ($C_1$-$C_4$)-Alkyl, ($C_2$-$C_4$)-Alkenyl, ($C_2$-$C_4$)-Alkinyl, Phenyl, Benzyl oder ($C_1$-$C_4$)-Alkoxy stehen und $R^7$ die für R genannte Bedeutung hat, bedeutet.

3. Mittel nach Anspruch 1 oder 2, dadurch gekennzeichnet, daß sie als Herbizide Wirkstoffe aus der Gruppe Carbamate, Thiocarbamate, Halogenacetanilide, substituierte Phenoxy-, Naphthoxy-, Phenoxyphenoxy-, Benzyloxyphenoxy- und Heteroaryloxyphenoxy-alkancarbonsäurederivate und Cyclohexandion-derivate enthalten.

4. Mittel nach Anspruch 3, dadurch gekennzeichnet, daß sie den Wirkstoff der Formel (I) oder deren Salze und das Herbizid im Gewichtsverhältnis von 10:1 bis 1:10 enthalten.

5. Verbindungen der nach Anspruch 1 oder 2 definierten Formel (I) oder deren Salze, ausgenommen solche Verbindungen, in denen X = Sauerstoffatom und

a) R = OH oder ($C_1$-$C_5$)-Alkoxy und $(Z)_n$ = einen einzelnen Rest in 2-, 3- oder 4-Position am Phenylring aus der Gruppe F, Cl, Br, CN, $OCHF_2$, $CF_3$ und $NO_2$ oder $(Z)_n$ = 3-Phenoxy, 2,3-, 2,4-, 2,5- oder 3,4-$Cl_2$ oder 2,4-, 3,4- oder 3,5-$Br_2$ oder 3-Cl-4-F, 5-Cl-2-$NO_2$ oder 2-Cl-5-$NO_2$ oder
b) R = $OCH_3$ und $(Z)_n$ = H, 3-$CH_3$, 4-$CH_3$, 2,6-, 4,6- oder 5,6-$Cl_2$, 4-$OCF_2Br$, 4-$OCF_3$, 4-Phenoxy, 4-Phenoxyphenyl, 2,4,6-$(OCH_3)_3$ oder 2,4,6-$(CH_3)_3$ oder
c) R = OH oder $OC_2H_5$ und $(Z)_n$ = H bedeuten.

6. Pflanzenschützende Mittel, dadurch gekennzeichnet, daß sie eine Verbindung der Formel (I) oder deren Salze noch Anspruch 5 und übliche Formulierungshilfsmittel enthalten.

7. Verfahren zur Herstellung von Verbindungen der Formel (I) oder deren Salzen nach Anspruch 5, dadurch gekennzeichnet, daß man ein Acrylsäurederivat der Formel (II)

$$H_2C = CH - CO - R \qquad (II)$$

mit einer Verbindung der Formel (III)

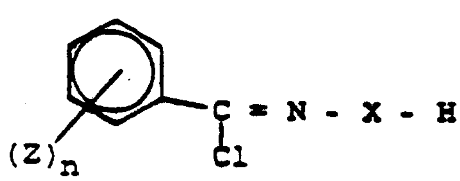

(III)

umsetzt, wobei in den Formeln R, Z, n und X die in Anspruch 5 genannten Bedeutungen haben.

8. Verfahren zur Bekämpfung von unerwünschten Pflanzen in Nutzpflanzenkulturen, dadurch gekennzeichnet, daß

man ein Herbizid in Kombination mit nach Anspruch 1 oder 2 definierten Verbindungen der Formel (I) oder deren Salzen auf die Pflanzen, Pflanzensamen oder die Anbaufläche appliziert.

9. Verfahren zum Schutz von Nutzpflanzen gegen phytotoxische Nebenwirkungen von Herbiziden, dadurch gekennzeichnet, daß man Herbizide in Kombination mit nach Anspruch 1 oder 2 definierten Verbindungen der Formel (I) oder deren Salzen auf die Pflanzen, Pflanzensamen oder die Anbaufläche appliziert.

10. Verwendung von nach Anspruch 1 oder 2 definierten Verbindungen der Formel (I) oder deren Salzen zum Schutz von Pflanzen gegen phytotoxische Nebenwirkungen von Herbiziden.

## Claims

1. A crop plant-protecting agent which contains a compound of the formula (I), or a salt thereof,

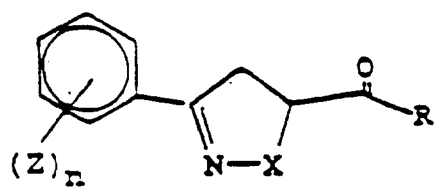

(I)

in which

X is an oxygen or sulfur atom,

R is OH, SH, alkoxy, alkenyloxy, alkynyloxy, alkylthio, alkenylthio, alkynylthio, cycloalkyloxy or cycloalkylthio, where the 8 last-mentioned groups are unsubstituted or mono- or polysubstituted by radicals from the group comprising phenyl, alkoxy, alkenyloxy, alkynyloxy, benzyloxy, phenyloxy, cycloalkyloxy, alkylthio, mono- and dialkylamino, cyano, halogen and $NO_2$,
or is benzyloxy, phenyloxy, benzylthio or phenylthio, where the 4 last-mentioned groups are unsubstituted or mono- or polysubstituted by radicals from the group comprising alkyl, alkenyl, alkynyl, halogen, cyano, $NO_2$, alkoxy, alkenyloxy, alkynyloxy, alkylthio, mono- and dialkylamino, phenyloxy and benzyloxy,
or is trialkylsilylalkoxy, aryldialkylsilyloxy, aralkyldialkylsilyloxy, diarylalkylsilyloxy, diaralkylalkylsilyloxy, a radical of the formula NR'R', in which the radicals R' are identical or different radicals from the group comprising alkyl, alkenyl, alkynyl and cycloalkyl, or is pyridino, morpholino, dialkylmorpholino and hydrazino, or is a radical of the formula

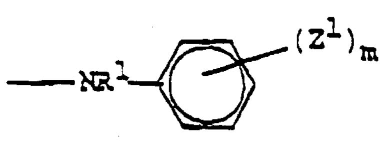

in which $R^1$ is a hydrogen atom, alkyl, alkenyl or alkynyl and the radicals $Z^1$ independently of one another are halogen, nitro, alkyl, alkenyl, alkoxy or phenoxy and m is an integer from 0 to 5, or
is a radical of the formula

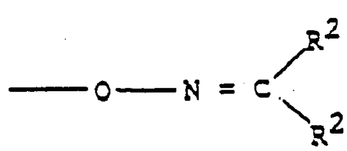

in which the radicals $R^2$ in each case independently of one another are alkyl, or together with the carbon atom linking them are cycloalkyl, or is

$$-O-CR^3R^3-CO-R^4$$

in which the $R^3$ radicals independently of one another are hydrogen, alkyl, alkenyl, alkynyl, aryl, benzyl, alkoxy, alkenyloxy, alkynyloxy or phenoxy and $R^4$ is hydrogen, alkyl, alkenyl, alkynyl, aryl or benzyl, or is a radical of the formula

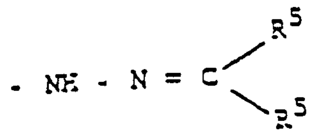

in which the $R^5$ radicals independently of one another are alkyl, hydrogen or aryl, or together with the carbon atom linking them are cycloalkyl, or is a radical of the formula

$$-O-CR^6R^6-CO-R^7$$

in which the radicals $R^6$ independently of one another are hydrogen, alkyl, alkenyl, alkynyl, aryl, benzyl, alkoxy, alkenyloxy, alkynyloxy or phenoxy and $R^7$ has the meaning given above for R,

Z is halogen, nitro, cyano, $(C_1-C_4)$alkyl, $(C_1-C_4)$alkoxy, $(C_1-C_4)$alkylthio, where the alkyl, alkoxy and alkylthio groups independently of one another are unsubstituted or substituted by one or more halogen atoms, or is $(C_3-C_6)$cycloalkyl which is unsubstituted or substituted by $(C_1-C_4)$alkyl, or is amino, hydroxymethyl, $(C_1-C_4)$alkylamino, di$(C_1-C_4)$alkylamino, $(C_1-C_4)$alkoxyethyl, where the alkyl and alkoxy groups in the 3 last-mentioned radicals independently of one another are unsubstituted or substituted by $(C_1-C_4)$alkyl, or is phenyl or phenoxy, where phenyl and phenoxy independently of one another are unsubstituted or mono- or polysubstituted by radicals from the group comprising halogen and trifluoromethyl, and

n is an integer from 0 to 5,

and one or more herbicides and, where appropriate, conventional formulation auxiliaries.

**2.** An agent as claimed in claim 1, in which

R is OH, SH, $(C_1-C_4)$alkoxy, $(C_2-C_4)$alkenyloxy, $(C_2-C_4)$alkynyloxy, $(C_1-C_4)$alkylthio, $(C_2-C_4)$alkenylthio, $(C_2-C_4)$alkynylthio, $(C_3-C_8)$cycloalkoxy or $(C_3-C_8)$cycloalkylthio, where the 8 last-mentioned groups are unsubstituted or mono- or polysubstituted by radicals from the group comprising phenyl, $(C_1-C_4)$alkoxy, $(C_2-C_4)$alkenyloxy, $(C_2-C_4)$alkynyloxy, benzyloxy, phenyloxy, $(C_3-C_8)$cycloalkyloxy, $(C_1-C_4)$alkylthio, mono- and di-$(C_1-C_4)$alkylamino, cyano, halogen and $NO_2$,
or is phenyloxy, phenylthio, benzyloxy or benzylthio, where the 4 last-mentioned groups are unsubstituted or substituted by 1 to 3 radicals from the group comprising $(C_1-C_4)$alkyl, $(C_2-C_4)$alkenyl, $(C_2-C_4)$alkynyl, halogen, cyano, $NO_2$, $(C_1-C_4)$alkoxy, $(C_2-C_4)$alkenyloxy, $(C_2-C_4)$alkynyloxy, $(C_1-C_4)$alkylthio, mono- and di-$(C_1-C_4)$alkylamino, phenyloxy and benzyloxy, or
is a radical of the formula -NR'R', in which R' is $(C_1-C_4)$alkyl, or is pyridino, morpholino, dimethylmorpholino or hydrazino, or is a radical of the formula

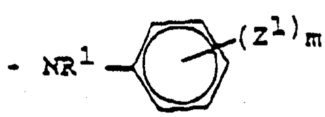

in which $R^1$ is hydrogen or $(C_1-C_4)$alkyl, the radicals $Z^1$ independently of one another are halogen, nitro, $(C_1-C_4)$alkyl, $(C_1-C_4)$alkoxy or phenoxy and m is 0 to 3, or
is a radical of the formula $-O-N=CR^2R^2$ in which the $R^2$ radicals are $(C_1-C_4)$alkyl, in particular methyl, or together with the carbon atom linking them are cyclohexyl or cyclopentyl,
or is a radical of the formula $-O-CR^3R^3-CO-R^4$ in which the $R^3$ radicals independently of one another are hydrogen, $(C_1-C_4)$alkyl, $(C_2-C_4)$alkenyl, $(C_2-C_4)$alkynyl, phenyl, benzyl or $(C_1-C_4)$alkoxy and $R^4$ is $(C_1-C_4)$alkyl, $(C_2-C_4)$alkenyl, $(C_2-C_4)$alkynyl, phenyl or benzyl, or
is a radical of the formula $-NH-N=CR^5R^5$ in which the $R^5$ radicals independently of one another are hydrogen, $(C_1-C_4)$alkyl or phenyl, or together with the carbon atom linking them are cyclohexyl or cyclopentyl,

or is a radical of the formula $-O-CR^6R^6-CO-R^7$ in which the $R^6$ radicals independently of one another are hydrogen, $(C_1-C_4)$alkyl, $(C_2-C_4)$alkenyl, $(C_2-C_4)$alkynyl, phenyl, benzyl or $(C_1-C_4)$alkoxy and $R^7$ has the meaning mentioned for R.

3. An agent as claimed in claim 1 or 2, which contains, as herbicide, an active ingredient from the group comprising carbamates, thiocarbamates, haloacetanilides, substituted phenoxy-, naphthoxy-, phenoxyphenoxy-, benzyloxyphenoxy- and heteroaryloxyphenoxyalkanecarboxylic acid derivatives and cyclohexanedione derivatives.

4. An agent as claimed in claim 3, which contains the active ingredient of the formula (I) or a salt thereof and the herbicide in a ratio by weight of from 10 : 1 to 1 : 10.

5. A compound of the formula (I), or a salt thereof, as defined in claim 1 or 2, with the exception of those compounds in which

X is an oxygen atom and

a) R is OH or $(C_1-C_5)$alkoxy and $(Z)_n$ is an individual radical in position 2, 3 or 4 on the phenyl ring from the group comprising F, Cl, Br, CN, $OCHF_2$, $CF_3$ and $NO_2$ or $(Z)_n$ is 3-phenoxy, 2,3-, 2,4-, 2,5-, or 3,4-$Cl_2$ or 2,4-, 3,4- or 3,5-$Br_2$ or 5-Cl-4-F, 5-Cl-2-$NO_2$ or 2-Cl-5-$NO_2$ or
b) R is $OCH_3$ and $(Z)_n$ is H, 3-$CH_3$, 4-$CH_3$, 2,6-, 4,6 or 5,6-$Cl_2$, 4-$OCF_2Br$, 4-$OCF_3$, 4-phenoxy, 4-phenoxyphenyl, 2,4,6-$(OCH_3)_3$ or 2,4,6-$(CH_3)_3$ or
c) R is OH or $OC_2H_5$ and $(Z)_n$ is H.

6. A plant-protecting agent which contains a compound of the formula (I) or a salt thereof as claimed in claim 5 and conventional formulation auxiliaries.

7. A process for preparing compounds of the formula (I) or salts thereof as claimed in claim 5, which comprises reacting an acrylic acid derivative of the formula (II)

$$H_2C = CH - CO - R \qquad (II)$$

with a compound of the formula (III)

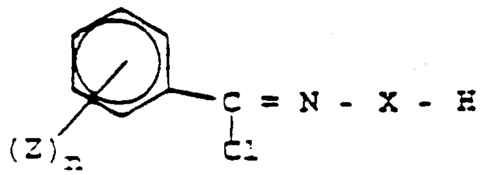

(III)

where R, Z, n and X in the formulae have the meanings mentioned in claim 5.

8. A method of controlling undesired plants in crops of useful plants, which comprises applying a herbicide in combination with compounds of the formula (I), or salts thereof, as defined in claim 1 or 2, to the plants, seeds of plants or the area under cultivation.

9. A method of protecting useful plants against phytotoxic side effects of herbicides, which comprises applying herbicides in combination with compounds of the formula (I), or salts thereof, as defined in claim 1 or 2, to the plants, seeds of plants or the area under cultivation.

10. The use of compounds of the formula (I), or salts thereof, as defined in claim 1 or 2, for protecting plants against phytotoxic side effects of herbicides.

**Revendications**

1. Agents phytoprotecteurs de plantes de culture caractérisés en ce qu'il contiennent un composé de formule (I) ou ses sels

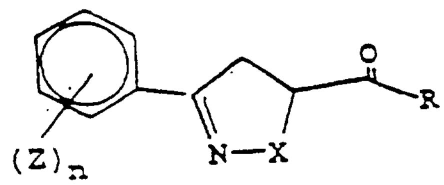

(I)

dans laquelle

X représente un atome d'oxygène ou de soufre,

R représente OH, SH, alcoxy, alcényloxy, alcynyloxy, alkylthio, alcénylthio, alcynylthio, cycloalkyloxy, cycloalkylthio,

les 8 derniers groupes cités étant non substitués ou substitués une ou plusieurs fois par des radicaux pris dans le groupe comportant phényle, alcoxy, alcényloxy, alcynyloxy, benzyloxy, phényloxy, cycloalkyloxy, alkylthio, mono- et dialkylamino, cyano, halogène et $NO_2$,

représente benzyloxy, phényloxy, benzylthio, phénylthio, ces 4 derniers groupes cités étant non substitués ou substitués une ou plusieurs fois par des radicaux pris dans le groupe comportant alkyle, alcényle, alcynyle, halogèno, cyano, $NO_2$, alcoxy, alcényloxy, alcynyloxy, alkylthio, mono-et dialkylamino, phényloxy et benzyloxy,

représente trialkylsilylalcoxy, aryldialkylsilyloxy, aralkyldialkylsilyloxy, diarylalkylsilyloxy, diaralkylalkylsilyloxy, un radical de formule NR'R', dans laquelle les radicaux R' représentent des radicaux identiques ou différents pris dans le groupe comportant alkyle, alcényle, alcynyle et cycloalkyle, pyridino, morpholino, dialkylmorpholino, hydrazino, ou

un radical de formule

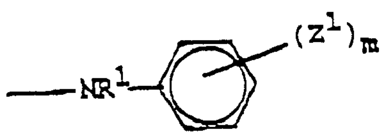

dans laquelle $R^1$ est un atome d'hydrogène, alkyle, alcényle ou alcynyle et les radicaux $Z^1$, indépendamment les uns des autres, représentent halogène, nitro, alkyle, alcényle, alcoxy ou phénoxy et m est un nombre entier de 0 à 5, ou un radical de formule

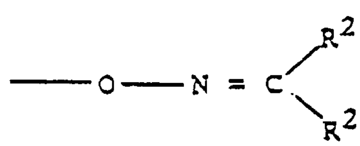

dans laquelle les radicaux $R^2$, chacun indépendamment de l'autre, représentent alkyle, ou ensemble avec l'atome de C qui les relie, représentent cycloalkyle, ou

$$- O - CR^3R^3 - CO - R^4$$

dans laquelle les radicaux $R^3$, indépendamment les uns des autres, représentent H, alkyle, alcényle, alcynyle, aryle, benzyle, alcoxy, alcényloxy, alcynyloxy ou phénoxy, et $R^4$ représente l'hydrogène, alkyle, alcényle, alcynyle, aryle ou benzyle, ou

un radical de formule

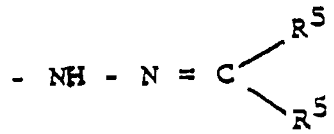

dans laquelle les radicaux $R^5$, indépendamment les uns des autres, représentent alkyle, H ou aryle, ou ensemble avec l'atome de C qui les relie représentent cycloalkyle, ou
un radical de formule

$$- O - CR^6R^6 - CO - R^7$$

dans laquelle les radicaux $R^6$, indépendamment l'un de l'autre, représentent H, alkyle, alcényle, alcynyle, aryle, benzyle, alcoxy, alcényloxy, alcynyloxy ou phénoxy et $R^7$ a la signification donnée précédemment pour R,

Z représente halogène, nitro, cyano, alkyle en $C_1$-$C_4$, alcoxy en $C_1$-$C_4$, (alkyl en $C_1$-$C_4$)thio, les groupes alkyle, alcoxy et alkylthio, indépendamment les uns des autres, étant non substitués ou substitués par un ou plusieurs atomes d'halogènes,
cycloalkyle en $C_3$-$C_6$, qui est non substitué ou substitué par alkyle en $C_1$-$C_4$, amino, hydroxyméthyle, (alkyl en $C_1$-$C_4$)amino, di(alkyl en $C_1$-$C_4$)amino, (alcoxy en $C_1$-$C_4$)méthyle, les groupes alkyle et alcoxy dans les trois derniers radicaux, indépendamment l'un de l'autre, étant non substitués ou substitués par alkyle en $C_1$-$C_4$, représente phényle ou phénoxy, phényle et phénoxy, indépendamment l'un de l'autre, étant non substitué ou substitué par un ou plusieurs radicaux pris dans le groupe comportant des halogènes et trifluorométhyle,

n est un nombre entier de 0 à 5, et un ou plusieurs herbicides ainsi qu'éventuellement des adjuvants de formulation usuels.

2. Agents selon la revendication 1, caractérisés en ce que

R représente OH, SH, alcoxy en $C_1$-$C_4$, (alcényl en $C_2$-$C_4$)oxy, (alcynyl en $C_2$-$C_4$)oxy, (alkyl en $C_1$-$C_4$)thio, (alcényl en $C_2$-$C_4$)thio, (alcényl en $C_2$-$C_4$)thio, cycloalcoxy en $C_3$-$C_8$, (cycloalkyl en $C_3$-$C_8$)thio, les 8 derniers groupes cités pouvant être non substitués ou substitués une ou plusieurs fois par des radicaux pris dans le groupe comportant phényle, alcoxy en $C_1$-$C_4$, (alcényl en $C_2$-$C_4$)oxy, (alcynyl en $C_2$-$C_4$)oxy, benzyloxy, phényloxy, cycloalcoxy en $C_3$-$C_8$, (alkyl en $C_1$-$C_4$)thio, mono- et di-(alkyl en $C_1$-$C_4$)amino, cyano, halogéno et $NO_2$,
représente phényloxy, phénylthio, benzyloxy, benzylthio, les quatre derniers groupes cités étant non substitués ou substitués par 1 à 3 radicaux pris dans le groupe comportant alkyle en $C_1$-$C_4$, alcényle en $C_2$-$C_4$, alcynyle en $C_2$-$C_4$, halogéno, cyano, $NO_2$, alcoxy en $C_1$-$C_4$, alcènyloxy en $C_2$-$C_4$, alcynyloxy en $C_2$-$C_4$, (alkyl en $C_1$-$C_4$)thio, mono- et di-(alkyl en $C_1$-$C_4$)amino, phényloxy et benzyloxy, ou
un radical de formule -NR'R', dans laquelle R' représente alkyle en $C_1$-$C_4$,
représente aussi pyridino, morpholino, diméthylmorpholino, hydrazino, ou un radical de formule

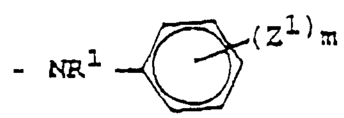

dans laquelle $R^1$ représente H ou alkyle en $C_1$-$C_4$, les radicaux $Z^1$, indépendamment les uns des autres, représentent halogéno, nitro, alkyle en $C_1$-$C_4$, alcoxy en $C_1$-$C_4$ ou phénoxy et m va de 0 à 3, ou
représente un radical de formule $-O-N=CR^2R^2$, dans laquelle $R^2$ représente alkyle en $C_1$-$C_4$, plus particulièrement méthyle, ou ensemble avec l'atome de C qui les relie, cyclohexyle ou cyclopentyle,
ou représente un radical de formule $-O-CR^3R^3-COR^4$, dans laquelle les radicaux $R^3$, indépendamment les uns des autres, représentent H, alkyle en $C_1$-$C_4$, alcènyle en $C_2$-$C_4$, alcynyle en $C_2$-$C_4$, phényle, benzyle ou alcoxy en $C_1$-$C_4$ et $R^4$ représente alkyle en $C_1$-$C_4$, alcènyle en $C_2$-$C_4$, alcynyle en $C_2$-$C_4$, phényle ou benzyle, ou
représente un radical de formule $-NH-N=CR^5R^5$, dans laquelle les radicaux $R^5$, indépendamment les uns des autres, représentent H, alkyle en $C_1$-$C_4$ oou phényle, ou ensemble avec l'atome de C qui les relie, représente cyclohexyle ou cyclopentyle ou un radical de formule -O-$CR^6R^6$-CO-$R^7$, dans laquelle les radicaux $R^6$, indépendamment l'un de l'autre, représentent H, alkyle en $C_1$-$C_4$, alcényle en $C_2$-$C_4$, alcynyle en $C_2$-$C_4$, phényle, benzyle ou alcoxy en $C_1$-$C_4$, et $R^7$ a la signification donnée pour R.

**3.** Agents selon la revendication 1 ou 2, caractérisés en ce qu'ils contiennent en tant que matière active herbicide pris dans le groupe comportant des carbamates, des thiocarbamates, des halogénacétanilides, des dérivés substitués de l'acide phénoxy-, naphtoxy-, phénoxyphénoxy-, benzyloxyphénoxy- et hétéroaryloxy-phénoxyalcanecarboxylique et des dérivés de cyclohexadione.

**4.** Agents selon la revendication 3, caractérisés en ce qu'ils contiennent la matière active de formule (I) ou ses sels et l'herbicide dans un rapport pondéral de 10:1 à 1:10.

**5.** Composés de formule (I) définis selon la revendication 1 ou 2, ou leurs sels, à l'exception des composés dans lesquels X représente l'oxygène et

a) R = OH ou alcoxy en $C_1$-$C_5$ et $(Z)_n$ = un seul radical en position 2, 3 ou 4 sur le cycle phényle pris dans le groupe comportant F, Cl, Br, CN, $OCHF_2$, $CF_3$ et $NO_2$ ou $(Z)_n$ = 3-phénoxy, 2,3-, 2,4-, 2,5- ou 3,4-$Cl_2$ ou 2,4-, 3,4- ou 3,5-$Br_2$ ou 3-Cl-4-F, 5-Cl-2-$NO_2$ ou 2-Cl-5-$NO_2$ ou
b) R = $OCH_3$ et $(Z)_n$ = H, 3-$CH_3$, 4-$CH_3$, 2,6-, 4,6- ou 5,6-$Cl_2$, 4-$OCF_2Br$, 4-$OCF_3$, 4-phénoxy, 4-phénoxyphényle, 2,4,6-$(OCH_3)_3$ ou 2,4,6-$(CH_3)_3$ ou
c) R = OH ou $OC_2H_5$ et $(Z)_n$ = H.

**6.** Agents phytoprotecteurs caractérisés en ce qu'ils contiennent un composé de formule (I) ou ses sels, selon la revendication 5 et des adjuvants de formulation usuels.

**7.** Procédé pour la préparation de composés de formule (I) ou de leurs sels selon la revendication 5, caractérisé en ce que l'on fait réagir un dérivé d'acide acrylique de formule (II)

$$H_2C = CH - CO - R \quad (II)$$

avec un composé de formule (III)

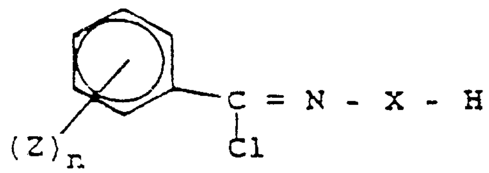

(III)

R, Z, n et X dans les formules ayant les significations données dans la revendication 5.

**8.** Procédé pour la lutte contre les plantes indésirables dans les cultures de plantes utiles, caractérisé en ce que l'on applique un herbicide en combinaison avec des composés de formule (I) définis dans la revendication 1 ou 2 ou leurs sels sur les plantes, les graines des plantes et les surfaces cultivables.

**9.** Procédé pour la protection de plantes de culture contre les effets secondaires phytotoxiques des herbicides, caractérisé en ce que l'on utilise les herbicides en combinaison avec les composés de formule (I) définis dans la revendication 1 ou 2 ou leurs sels sur les plantes, les graines des plantes et les surfaces cultivables.

**10.** Utilisation des composés de formule (I) définis selon la revendication 1 ou 2, ou de leurs sels pour la protection de plantes contre les effets indésirables phytotoxiques des herbicides.